# EUROPEAN PATENT APPLICATION

(11) **EP 4 621 042 A1**
(43) Date of publication of application: **24.09.2025**
(21) Application number: 23890863.6
(22) Date of filing: 16.11.2023
(51) Int. Cl.: C12M 3/00, C12M 1/38, C12M 1/36, C12M 1/04, C12M 1/00

(54) **SYSTEM FOR PRODUCING TARGET SUBSTANCE, AND METHOD**

(30) Priority: 16.11.2022 CN 202211437349
(71) Applicant: Oxford University (Suzhou) Science & Technology Co., Ltd., Suzhou, Jiangsu 215123 (CN)
(72) Inventor: LEI, Rui, Suzhou, Jiangsu 215123 (CN); ZENG, Yida, Suzhou, Jiangsu 215123 (CN); YE, Hua, Suzhou, Jiangsu 215123 (CN); CUI, Zhanfeng, Suzhou, Jiangsu 215123 (CN)
(74) Representative: HGF
(86) International application number: PCT/CN2023/132083
(87) International publication number: WO 2024/104434

(57) **Abstract**

The present application relates to a system and a method for producing a target substance. The system comprises a culturing device, a first culturing solution supply device, a second culturing solution supply device, and a culture substance supply unit. The culturing device is configured to be divided by a filter membrane into a growth zone and a culturing solution circulation zone; the first culturing solution supply device is configured to be in fluid communication with the growth zone via a first inlet and a first outlet; the second culturing solution supply device is configured to be in fluid communication with the culturing solution circulation zone via a second inlet and a second outlet; and the culture substance supply unit is configured to be in fluid communication with the growth zone via the first inlet. The system can obtain a target substance at a higher yield in a simple and efficient process.

## Description

### RELATED APPLICATIONS

This application claims priority to Chinese patent application No. 2022114373492, filed on November 16, 2022, and entitled "SYSTEM AND METHOD FOR PRODUCING TARGET SUBSTANCE", which is hereby incorporated by reference in its entirety.

### TECHNICAL FIELD

The present disclosure relates to the field of biotechnology, and in particular to a system and method for producing a target substance.

### BACKGROUND

The production of target substances usually involves a process of rapid growth of the target substances with the help of artificially created culture conditions, such as suitable culture medium and culture temperature, etc. Taking cell culture as an example, cells are incubated and cultured under specific *in vitro* conditions such as simulating the physiological environment in a living organism, so that the cells can survive and proliferate. In order for smooth grow of cells, it is often necessary to detect the growth environment of cells during cell culture. According to conventional cell culture, cells are generally inoculated in a reactor, in which culture medium for growth of cells or tissues is fed through a delivery pipeline.

Currently, flasks are often used as culture devices for the production of cells or their target metabolites, e.g., extracellular vesicle (EV). The defects include that the culture device occupies a substantial amount of space, and the concentration of cells or their target metabolites in the resulting culture solution is excessively low, requiring complex downstream processing to concentrate the produced cells or their target metabolites. Moreover, this culture method, due to the need for complex manual operations, pose the possibility of contaminating the cell culturing solution, and fails to guarantee the batch-to-batch consistency of the products. In addition, the current production process has defects including a small surface area to volume ratio, material transfer only by diffusion, local adverse conditions, and waste accumulation.

Therefore, there is still an urgent need for systems and methods for obtaining high-content, low-pollution target substances in a simple and efficient manner.

### SUMMARY

An object of embodiments of the present disclosure is to provide a system and method for producing a target substance.

In one aspect, the present disclosure provides a system for producing a target substance, the system including:
a. a culture device configured to be divided into a growth zone and a culturing solution circulation zone via a filter membrane, and including a first inlet, a first outlet, a second inlet and a second outlet,
b. a first culturing solution supply device configured to be in fluid communication with the growth zone via the first inlet and the first outlet,
c. a second culturing solution supply device configured to be in fluid communication with the culturing solution circulation zone via the second inlet and the second outlet, and
d. a culture substance supply unit configured to be in fluid communication with the growth zone via the first inlet.

In an embodiment of the present disclosure, the system further includes:
e. a plurality of perfusion pumps disposed between the culture device and the first culturing solution supply device and/or disposed between the culture device and the second culturing solution supply device.

In an embodiment of the present disclosure, a first valve is disposed between the first culturing solution supply device and the first inlet, and a second valve is disposed between the first culturing solution supply device and the first outlet.

In an embodiment of the present disclosure, a third valve is disposed between the second culturing solution supply device and the second inlet, and a fourth valve is disposed between the second culturing solution supply device and the second outlet.

In an embodiment of the present disclosure, the culture substance supply unit is in fluid communication with the first culturing solution supply device via a fifth valve, and
the first culturing solution supply device is in fluid communication with the second culturing solution supply device via a sixth valve.

In an embodiment of the present disclosure, the system of the present disclosure further includes a plurality of sensors, wherein the sensors are configured to perform sensing, for example, sense temperature, concentration, flow rate, etc. of the fluid in a flow path between the culture device and the first culturing solution supply device, for example, arranged in a first channel between the culture device and the first culturing solution supply device, and/or
the sensors are configured to perform sensing, for example, sense temperature, concentration, flow rate, etc. of the fluid in a flow path between the culture device and the second culturing solution supply device, for example, arranged in a second channel between the culture device and the second culturing solution supply device.

In the embodiments of the present disclosure, the sensor can be one or more selected from a pressure sensor, a temperature sensor, a dissolved oxygen sensor, a pH sensor, and a bubble detector.

In an embodiment of the present disclosure, the system of the present disclosure further includes an oxygenator. The oxygenator is configured to supply oxygen to a channel between the second culturing solution supply device and the culture device, for example, arranged in the second channel between the second culturing solution supply device and the culture device.

In an embodiment of the present disclosure, the system of the present disclosure further includes a plurality of sampling units, wherein the plurality of sampling units are in fluid communication with one or more of the first inlet, the first outlet, the second inlet or the second outlet.

In an embodiment of the present disclosure, the system of the present disclosure further includes a waste collection unit, wherein the waste collection unit is configured to be in fluid communication with the culture device via the first outlet or the second outlet.

In an embodiment of the present disclosure, the first culturing solution supply device includes a first culture medium supply unit and a first pretreatment solution supply unit, and/or
the second culturing solution supply device includes a second culture medium supply unit and a second pretreatment solution supply unit.

In an embodiment of the present disclosure, the first culture medium supply unit can be configured to store a first culture medium, and the first pretreatment solution supply unit can be configured to store a first pretreatment solution. The second culture medium supply unit can be configured to store a second culture medium, and the second pretreatment solution supply unit can be configured to store a second pretreatment solution. The first culture medium and the second culture medium can be the same or different, and the first pretreatment solution and the second pretreatment solution can be the same or different.

In an embodiment of the present disclosure, the filter membrane can be a microfiltration membrane, an ultrafiltration membrane or a nanofiltration membrane. In a specific embodiment, the filter membrane is a hollow fiber tube.

In a specific embodiment, the growth zone is an outer space of the hollow fiber tube, and the culturing solution circulation zone is an inner space of the hollow fiber tube.

In a specific embodiment, the growth zone is an inner space of the hollow fiber tube, and the culturing solution circulation zone is an outer space of the hollow fiber tube.

In an embodiment of the present disclosure, a surface of the filter membrane corresponding to the growth zone is treated with a coating solution.

In an embodiment of the present disclosure, the coating solution is an aqueous solution or a salt buffer solution containing one or more of fibronectin, vitronectin, osteopontin, collagen, gelatin, laminin or polylysine.

In another aspect, the present disclosure provides a method for producing a target substance, the method including:
a) supplying a culture substance to a growth zone of a culture device;
b) supplying a first culturing solution to the growth zone; and
c) supplying a second culturing solution to a culturing solution circulation zone of the culture device, wherein the culturing solution circulation zone is separated from the growth zone via a filter membrane.

In an embodiment of the present disclosure, the method includes washing the culture device with a pretreatment solution before step a).

In a specific embodiment, the pretreatment solution can be an aqueous solution or a salt buffer solution containing one or more of fibronectin, vitronectin, osteopontin, collagen, gelatin, laminin or polylysine.

In an embodiment of the present disclosure, the method further includes: subjecting the culture device to an inversion treatment to achieve sufficient adhesion of the culture substance, such as cells, after step a) and before step b). In a specific embodiment, the inversion treatment includes inverting the culture device 6 to 10 times at a frequency of 20 to 40 minutes per time.

In an embodiment of the present disclosure, the first culturing solution is supplied to the growth zone at a flow rate of 0.05 to 0.30 mL/min.

In an embodiment of the present disclosure, the second culturing solution is supplied to the culturing solution circulation zone at a flow rate of 50 to 500 mL/min.

In an embodiment of the present disclosure, during the culture period of the cells, a concentration of the target substance in the growth zone and contents of lactic acid and glucose in the growth zone and the culturing solution circulation zone are detected at a frequency of 1 to 4 days/time, and/or a pH value, dissolved oxygen and intracavitary pressure in the growth zone and the culturing solution circulation zone are monitored online in real time.

In the embodiments of the present disclosure, the culture substance includes but is not limited to bacteria, adherent cells such as mesenchymal stem cells, fibroblasts, endothelial cells, etc., or suspended cells such as immune cells, hematopoietic stem cells, neural stem cells, etc. The culture substance can also be a microcarrier loaded with cells or encapsulated with cells.

In an embodiment of the present disclosure, the target substance can be the culture substance itself or a metabolite of the culture substance, including but being not limited to cells, extracellular vesicles, viruses, vaccines, antibodies, proteins and other metabolites. In a specific embodiment of the present disclosure, the target substance is exosomes.

In the embodiments of the present disclosure, the culture period can vary depending on the target substance being cultured. For example, the culture period can be about several minutes to several days, or even several weeks, for example, about 30 minutes, about 1 hour, about 5 hours, about 10 hours, about 15 hours, about 24 hours, about 2 to 13 days, for example, about 3 days, 4 days, 5 days, 6 days, 7 days, 8 days, 9 days, 10 days, 11 days, 12 days or 13 days. In a specific embodiment, the culture period is 7 days. In a specific embodiment, the culture period is 9 days. In a specific embodiment, the culture period is 11 days.

In a specific embodiment of the present disclosure, the cells are inoculated into the growth zone at a density of about 1×10⁶ cells/ml to 1×10⁸ cells/ml, for example, about 1×10⁶ to 5×10⁶ cells/ml, for example, about 1×10⁶ to 5×10⁷ cells/ml, for example, about 1×10⁶ cells/ml, for example, about 5×10⁶ cells/ml, for example, about 1×10⁷ cells/ml, for example, about 5×10⁷ cells/ml.

The system and method of the present disclosure can be used to culture any substance suitable for culturing in the system, not limited to specific mesenchymal stem cells, which include human mesenchymal stem cells, animal mesenchymal stem cells, etc.

Additional aspects and advantages of the present invention will be given in part in the following descriptions, become apparent in part from the following descriptions, or be learned from the practice of the present invention.

### BRIEF DESCRIPTION OF THE DRAWINGS

Other aspects and advantages of the present disclosure will become more apparent from the following description, taken in conjunction with the exemplary drawings, which are provided for illustrative purposes only and are not to be construed as limiting, wherein:
FIG. 1 shows a system for producing a target substance according to an embodiment of the present disclosure.
FIG. 2 shows a schematic diagram of the system for producing a target substance according to a specific embodiment of the present disclosure.

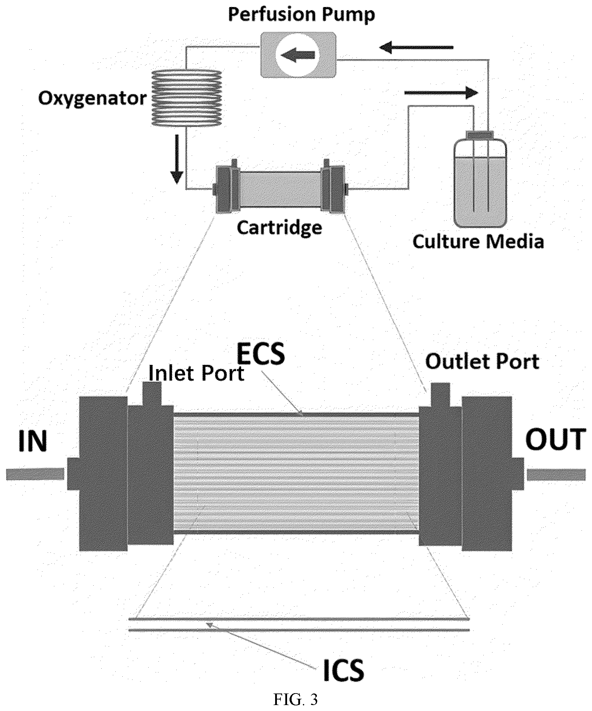

FIG. 3 shows a schematic structural diagram of the system for producing a target substance according to an embodiment of the present disclosure.
FIG. 4 shows a flow chart of a method for producing a target substance according to an embodiment of the present disclosure.
FIG. 5 shows a flow chart of the method for producing a target substance according to another embodiment of the present disclosure.
FIG. 6 shows a graph of the total number of viable cells harvested using a flask and the system of the present disclosure.
FIG. 7 shows a graph showing the cell expansion fold relative to the starting cell number of viable cells harvested using a flask and the system of the present disclosure.
FIG. 8 shows a graph of the viability of viable cells relative to the total cell number harvested using a flask and the system of the present disclosure.
FIG. 9 shows a graph showing the exosome yield (expressed as protein amount) per million cells harvested using a flask and the system of the present disclosure.
FIG. 10 shows a graph of the exosome yield (expressed as protein amount) harvested per 48 hours using a flask and the system of the present disclosure.
FIG. 11 shows a graph showing the exosome yield (expressed as particle numbers) per million cells harvested using a flask and the system of the present disclosure.
FIG. 12 shows a graph of the exosome yield (expressed as particle numbers) harvested per 48 hours using a flask and the system of the present disclosure.
FIG. 13 shows a graph showing the migratory effect of the exosomes harvested using a flask and the system of the present disclosure on fibroblasts.
FIG. 14 shows a graph showing the promotion effect of the exosomes harvested using a flask and the system of the present disclosure on fibroblast proliferation.

### DETAILED DESCRIPTION

A detailed description of embodiments of the present disclosure will be given below. Illustrative embodiments are shown in the drawings, in which the similar reference signs are used throughout to represent the same or similar elements or elements having the same or similar functions. The embodiments described with reference to the drawings are illustrative, which are merely used to interpret the present disclosure, but cannot be understood as limitation to the present disclosure.

In the related art, flasks are usually used as cell reactors to produce target substances, such as extracellular vesicle (EV). Flask-based production methods have the defects of small surface area to volume ratio, large incubator space, material transfer only by diffusion, local adverse conditions, and waste accumulation. The harvested cell culture solution usually contains a large amount of culture medium, and extremely low concentration of cells or their target metabolites, often requiring complex purification treatments to concentrate the product, thereby resulting in yield loss.

In order to solve the above technical problems, embodiments of the present disclosure provide an improved system for producing a target substance. Embodiments of the present disclosure also provide a method for producing a target substance using the system of the present disclosure. The system and method of the present disclosure can obtain a higher yield of a target substance, such as a target cell or its target metabolite, in a simple and efficient manner.

Specifically, the system of the present disclosure is a bioreactor based on a filter membrane. The filter membrane can be a microfiltration membrane, an ultrafiltration membrane or a nanofiltration membrane. The filter membrane is specifically a hollow fiber tube. By utilizing the hollow fiber tube, the culture is allowed to adhere to and grow on the hollow fiber membrane, thereby maximizing the space utilization efficiency of the bioreactor, allowing efficient production of the target substance, such as target cells or their target metabolites.

According to the system of the present disclosure, the culturing solution used to culture the culture substance is circulated, so that the waste generated by cell metabolism can be taken away in a timely and sufficient manner as the culturing solution flows, allowing the culture substance to have a high metabolic activity during the culture period, thereby ensuring efficient production of target metabolites. More specifically, utilizing the system, a first culture medium is supplied to a growth zone at a relatively low flow rate, and a second culture medium is supplied to a culturing solution circulation zone at a relatively high flow rate, thereby, based on the flow rate difference, supplying the nutrients and oxygen required by the culture substance to the growth zone through the membrane, and taking away the metabolic waste, while ensuring the stable growth of the culture substance.

Further, the system is closed during production and performs an automated production process, thereby substantially reducing contamination caused by the external environment or human operation and improving batch consistency of the product.

Furthermore, the filter membrane structure of the system can concentrate the culture substance and the target metabolites produced in the growth zone, thereby achieving a concentration effect, effectively reducing downstream purification treatments and simplifying the production process.

Furthermore, the system provides two sets of culturing solution supply devices independently for the region inside the filter membrane, specifically the inner space of the hollow fiber tube, and the region outside the filter membrane, specifically the outer space of the hollow fiber tube, so as to flexibly and in real time adjust the culturing solution based on the state of the culture substance cultured in the growth zone, thereby ensuring the optimal culture conditions, thereby accelerating the growth of the culture substance, and then obtaining a higher growth rate and a higher yield of target substances, such as target cells or their target metabolites.

In addition, compared with a system using only one set of culturing solution supply device, the system using two sets of culturing solution supply devices in the present disclosure can more sensitively and independently adjust the culture conditions of the growth zone and the culturing solution circulation zone, thereby ensuring better culture conditions and obtaining a higher growth rate and higher yield.

The system and method for producing a target substance provided in the embodiments of the present disclosure are described in detail below with reference to the accompanying drawings.

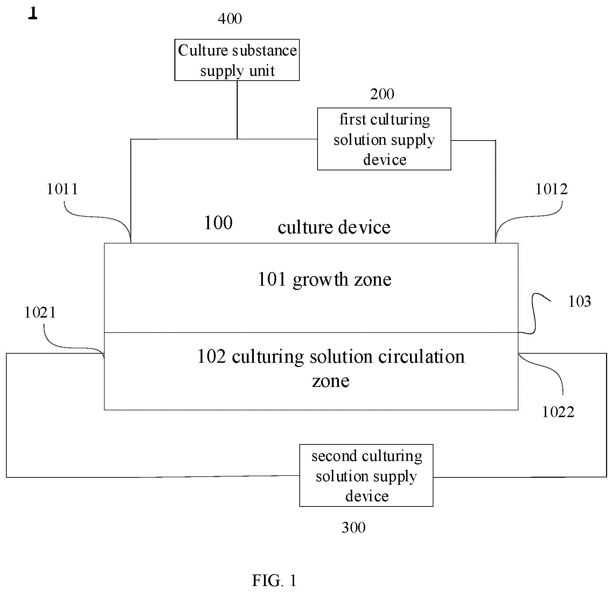

FIG. 1 shows a system for producing a target substance according to an embodiment of the present disclosure.

Referring to FIG. 1, the system 1 includes a culture device 100, a first culturing solution supply device 200, a second culturing solution supply device 300, and a culture substance supply unit 400. The culture device 100 includes a growth zone 101 and a culturing solution circulation zone 102. The culture device 100 is divided into the growth zone 101 and the culturing solution circulation zone 102 via a filter membrane 103.

The first culturing solution supply device 200 is configured to be in fluid communication with the growth zone 101 via the first inlet 1011 and the first outlet 1012 and to supply a first culturing solution to the growth zone 101.

The second culturing solution supply device 300 is configured to be in fluid communication with the culturing solution circulation zone 102 via the second inlet 1021 and the second outlet 1022 and to supply a second culturing solution to the culturing solution circulation zone 102.

The culture substance supply unit 400 is configured to be in fluid communication with the growth zone 101 via the first inlet 1011 and to supply the culture substance to the growth zone 101.

In an embodiment of the present disclosure, the system 1 can further include a plurality of perfusion pumps. The plurality of perfusion pumps are disposed between the culture device 100 and the first culturing solution supply device 200 and/or between the culture device 100 and the second culturing solution supply device 300, and are configured to pump the culture substance, the first culturing solution, and the second culturing solution to the culture device 100, respectively. By using the perfusion pumps, the system can realize the circulation of the cell culturing solution.

According to a specific embodiment, the filter membrane is a hollow fiber tube. Hereinafter, the culture device formed by using the hollow fiber tube is referred to as a hollow fiber tube reactor. By utilizing the hollow fiber tube, the culture substance is allowed to adhere to and grow on the hollow fiber membrane, thereby improving the space utilization efficiency of the hollow fiber tube reactor, allowing efficient production of the target substance.

According to an embodiment of the present disclosure, the hollow fiber tube has a pore size of not more than about 500 nm. According to an embodiment of the present disclosure, the hollow fiber tube has a pore size of about 20 nm to 200 nm, such as 20 nm to 180 nm, such as 20 nm to 170 nm, such as 20 nm to 160 nm, such as 20 nm to 150 nm, such as 20 nm to 140 nm, such as 20 nm to 130 nm, such as 20 nm to 120 nm, such as 20 nm to 110 nm, such as 20 nm to 100 nm, such as 20 nm to 90 nm, such as 20 nm to 80 nm, such as 20 nm to 70 nm, such as 20 nm to 60 nm, such as 20 nm to 50 nm, such as 20 nm to 40 nm. In a specific embodiment, the hollow fiber tube has a pore size of not more than 40 nm. In a specific embodiment, the hollow fiber tube has a pore size of not more than about 80 nm. In a specific embodiment, the hollow fiber tube has a pore size of not more than about 100 nm. In a specific embodiment, the hollow fiber tube has a pore size of not more than about 120 nm. In a specific embodiment, the hollow fiber tube has a pore size of not more than about 150 nm. In other embodiments, the pore size of the hollow fiber tube can vary according to the size of the target substance desired to be produced.

The growth zone can be configured to be the outer space of the hollow fiber tube. The culturing solution circulation zone can be configured to be the inner space of the hollow fiber tube.

In an alternative embodiment, the growth zone can be configured to be the inner space of the hollow fiber tube, and the culturing solution circulation zone can be configured to be the outer space of the hollow fiber tube.

As required, a plurality of hollow fiber tubes can be arranged in the hollow fiber tube reactor so as to have sufficient growth space, thereby obtaining a large amount of proliferated target substances, such as target cells or their target metabolites, in a confined environmental space.

According to the embodiment of the present disclosure, the culture substance or the first culturing solution is pumped to the growth zone by a perfusion pump. The second culturing solution is pumped to the culturing solution circulation zone by a perfusion pump. In a specific embodiment, the perfusion pump is a peristaltic pump, which can generate a pulse flow of the culturing solution in the culturing solution circulation channel, so that the culturing solution in the culturing solution circulation zone and the culture substance in the growth zone can achieve a relatively high material exchange efficiency.

According to the embodiment of the present disclosure, a first valve is disposed between the first culturing solution supply device and the first inlet corresponding to the growth zone. A second valve is disposed between the first culturing solution supply device and the first outlet corresponding to the growth zone. A third valve is disposed between the second culturing solution supply device and the second inlet corresponding to the culturing solution circulation zone. A fourth valve is provided between the second culturing solution supply device and the second outlet corresponding to the culturing solution circulation zone.

In the embodiments of the present disclosure, the culturing solution supplied to the growth zone and the culturing solution supplied to the culturing solution circulation zone can be independently controlled by adopting the specific valve setting. The circulation of the culturing solution can also be adjusted by using the valve setting. By controlling the opening and closing of the valves, the pressure difference between the growth zone and the culturing solution circulation zone can be adjusted, thereby more flexibly controlling the material exchange between the growth zone and the culturing solution circulation zone.

By using the valves, the culture substance, such as cells, can adhere to the hollow fiber membrane more evenly. Specifically, after the prepared culture is inoculated into the growth zone and before the automatic culture mode is turned on, the valves corresponding to both ends of the growth zone are closed, and the hollow fiber tube reactor is inverted at a specific time interval, so that the culture substance can be more evenly adhered.

Specifically, the inversion treatment includes inverting the culture device 6 to 10 times at a frequency of 20 to 40 minutes per time, for example 30 minutes per time. Thus, the aggregation and death of the culture substance, such as cells, is significantly reduced, thereby advantageously promoting the culture substance to grow at a relatively high growth rate and efficiently producing target substances, such as target cells or their target metabolites.

In an embodiment of the present disclosure, the culture substance supply unit is configured to be in fluid communication with the first culturing solution supply device via a fifth valve. Thus, a culture substance suspension and the culturing solution can be independently supplied to the growth zone of the hollow fiber tube reactor as needed.

In an embodiment of the present disclosure, the first culturing solution supply device for supplying the culturing solution to the growth zone is in fluid communication with the second culturing solution supply device for supplying the culturing solution to the culturing solution circulation zone via a sixth valve. Thus, the supply manner for supplying the culturing solution to the hollow fiber tube can be more flexibly adjusted.

According to the above embodiments of the present disclosure, the culturing solution for culturing the culture substance in the system is circulated, the first culturing solution is supplied to the growth zone at a relatively low flow rate of about 0.05 to 0.30 mL/min, and the second culturing solution is supplied to the culturing solution circulation zone at a relatively high flow rate of about 50 to 500 mL/min. The metabolic waste generated by metabolism of the culture substance in the growth zone can be taken away in a timely and sufficient manner as the culturing solution flows, allowing the culture substance to have a high metabolic activity during the culture period, thereby ensuring efficient production. Further, the system is closed during production and performs an automated production process, thereby substantially reducing contamination caused by the external environment or human operation, and improving batch consistency of the product.

In an embodiment of the present disclosure, the system of the present disclosure further includes a plurality of sensors. The sensors are configured to perform sensing in the flow path between the culture device and the first culturing solution supply device, for example, arranged in a first channel between the culture device and the first culturing solution supply device. In another embodiment, the sensors are configured to perform sensing in the flow path between the culture device and the second culturing solution supply device, for example, arranged in a second channel between the culture device and the second culturing solution supply device. The sensor can be one or more selected from a pressure sensor, a temperature sensor, a dissolved oxygen sensor, a pH sensor, and a bubble detector.

According to the embodiments of the present disclosure, by adjusting the pressure difference between the culturing solution circulation zone and the growth zone, combined with the specific pore size of the hollow fiber tube, the culture substance and its target metabolites produced can be concentrated in the growth zone, thereby achieving a concentration effect, thus effectively reducing downstream purification treatments and simplifying the production process.

In an embodiment of the present disclosure, the system of the present disclosure further includes an oxygenator. The oxygenator is configured to add oxygen to the culturing solution so as to provide sufficient oxygen for the culture substance in the growth zone. In a specific embodiment, the oxygenator is configured to add oxygen to the second channel between the second culturing solution supply device and the culture device so as to replenish oxygen in time as needed. Oxygen is transferred from the culturing solution circulation zone to the growth zone via the filter membrane. By adding gas to the system, the oxygenator can also adjust the pressure in the growth zone, thereby adjusting the pressure difference between the culturing solution circulation zone and the growth zone within a suitable range, and further controlling the material exchange between the growth zone and the culturing solution circulation zone.

The "first channel" and the "second channel" in the present disclosure are components configured to enable various devices or units to be in fluid communication, such as delivery pipes. Their structures and functions are well known in the art and will not be described in detail here.

In an embodiment of the present disclosure, the system of the present disclosure further includes a sampling unit. The system includes a plurality of sampling units. The plurality of sampling units are configured to be in fluid communication with one or more of the first inlet, the first outlet, the second inlet, or the second outlet. The system of the present disclosure can include 2 to 8 sampling units. For example, the system of the present disclosure can include 4 sampling units. Specifically, the sampling unit is disposed adjacent to the first inlet 1011 corresponding to the growth zone 101. Another sampling unit is disposed adjacent to the first outlet 1012 corresponding to the growth zone 101. Another sampling unit is disposed adjacent to the second inlet 1021 corresponding to the culturing solution circulation zone 102. A yet another sampling unit is disposed adjacent to the second outlet 1022 corresponding to the culturing solution circulation zone 102.

By using the sampling unit, the culturing solution in the culturing solution circulation zone can be sampled, and then the lactic acid content and glucose content therein can be detected, thereby monitoring the quality of the culturing solution in the system. By using the sampling unit, the culturing solution in the growth zone can be sampled, and then the lactic acid content and glucose content therein, the number of the cultured substance or the protein amount and particle number of the target metabolites can be detected, thereby monitoring the culture process in real time and accurately. Further, after the culturing process is completed, the target substance concentrated in the growth zone is collected by using the sampling unit.

In an embodiment of the present disclosure, the system of the present disclosure further includes a waste collection unit. The waste collection unit can be configured to be in fluid communication with the culture device via the first outlet or the second outlet, and configured to collect waste liquid discharged from the culture device.

In a specific embodiment, the sampling unit is disposed between the culture device and the waste collection unit, thereby determining whether the nutrients in the circulating culturing solution have been fully utilized. In a specific embodiment, one or more valves are arranged between the culture device and the waste collection unit, thereby controlling the culturing solution in the growth zone or the culturing solution in the culturing solution circulation zone to be recycled or discharged as waste liquid.

In an embodiment of the present disclosure, the first culturing solution supply device includes a first culture medium supply unit for accommodating a first culture medium, and a first pretreatment solution supply unit for accommodating a first pretreatment solution. Thus, nutrients required by the culture substance can be conveniently supplied to the growth zone through a pipeline. At the same time, the pretreatment solution, such as a buffer solution, can also be supplied to the growth zone as needed to clean the growth zone. In a specific embodiment, there are a plurality of first culture medium supply units. The first culture medium can include a culture medium for culturing the culture substance, such as a cell culture medium. Therefore, the first culturing solution supply device can include a supply unit for accommodating the pretreatment solution, and a supply unit for accommodating the cell culture medium.

In a specific embodiment, the pretreatment solution includes a culturing solution for washing the growth zone, such as a buffer solution, for example sterile water, physiological saline, phosphate buffered saline (PBS), and the like.

The pretreatment solution can also be a coating solution. The surface of the filter membrane corresponding to the growth zone is treated with the coating solution to enhance the adhesion ability of the culture substance such as cells. In a specific embodiment of the present disclosure, the coating solution is an aqueous solution or a salt buffer solution containing one or more of fibronectin, vitronectin, osteopontin, collagen, gelatin, laminin or polylysine.

In the embodiments of the present disclosure, the cell culturing solution is the most suitable culture medium for culturing cells. In a specific embodiment, the culture substance includes but is not limited to mesenchymal stem cells (MSCs). Therefore, the cell culturing solution includes but is not limited to MSC culture medium.

In the present disclosure, the system can be used to culture any substance that is suitably cultured in the system.

In the embodiments of the present disclosure, the culture substance includes but is not limited to bacteria, adherent cells such as mesenchymal stem cells, fibroblasts, endothelial cells, etc., or suspended cells such as immune cells, hematopoietic stem cells, neural stem cells, etc. The culture substance can also be a microcarrier loaded with cells or encapsulated with cells.

In an embodiment of the present disclosure, the second culturing solution supply device includes a second culture medium supply unit and a second pretreatment solution supply unit. Thus, the culturing solution can be conveniently supplied to the culturing solution circulation zone through a pipeline. The first culture medium and the second culture medium can be the same or different. The first pretreatment solution and the second pretreatment solution can be the same or different.

In a specific embodiment, there are a plurality of second culture medium supply units. For example, the second culture medium can include, but is not limited to, Dulbecco's Minimum Essential Medium (DMEM) culture medium, MSC culture medium, cell growth promoting factors, fetal bovine serum, sugars, amino acids, vitamins, inorganic ions and trace elements, etc. The second pretreatment solution includes, but is not limited to, sterile water, normal saline, phosphate buffer saline (PBS), 4-hydroxyethylpiperazineethanesulfonic acid (HEPES) buffer, tris(hydroxymethylaminomethane) (Tris) buffer, etc. Therefore, the second culturing solution supply device can include a plurality of second culture medium supply units and a plurality of second pretreatment solution supply units to independently supply nutrients to the culturing solution circulation zone. In an embodiment of the present disclosure, valves can be independently disposed at the outlet of each culture medium supply unit to independently supply the required nutrients to the culture device.

In an embodiment of the present disclosure, the first culturing solution supply device can include, in addition to the first culture medium supply unit and the first pretreatment solution supply unit, a plurality of units that facilitate the circulation of the first culturing solution in the growth zone, such as a liquid storage unit, a gas exchange unit, etc. Specifically, the liquid storage unit is configured to store the solution output from the first culture medium supply unit or the first pretreatment solution supply unit, or store the culturing solution flowing out from the growth zone.

In an embodiment of the present disclosure, the second culturing solution supply device can include, in addition to the second culture medium supply unit and the second pretreatment solution supply unit, a plurality of units that facilitate the circulation of the second culturing solution in the culturing solution circulation zone, such as a liquid storage unit, a gas exchange unit, etc.

In the embodiments of the present disclosure, the culture substance includes but is not limited to animal cells. In a specific embodiment, the culture substance includes but is not limited to bacteria, adherent cells such as mesenchymal stem cells, fibroblasts, endothelial cells, etc., or suspended cells such as immune cells, hematopoietic stem cells, neural stem cells, etc. The culture substance can also be a microcarrier loaded with cells or encapsulated with cells.

In an embodiment of the present disclosure, the target substance can include the culture substance itself or a metabolite of the culture substance, including but not limited to cells, extracellular vesicles, viruses, vaccines, antibodies, proteins and other metabolites. In a specific embodiment, the target substance is an exosome.

In an embodiment of the present disclosure, the extracellular vesicles are secreted by cells, and the hollow fiber tube reactor provides a substrate for cell growth. The porous hollow fiber membrane allows cell culturing materials and water to enter the growth zone through the membrane, while preventing the cells and extracellular vesicles with relatively large sizes from flowing out of the growth zone, thereby achieving the concentration of extracellular vesicles. In the case where the target metabolite is exosomes, the pore size of the hollow fiber membrane is set to less than 40 nm.

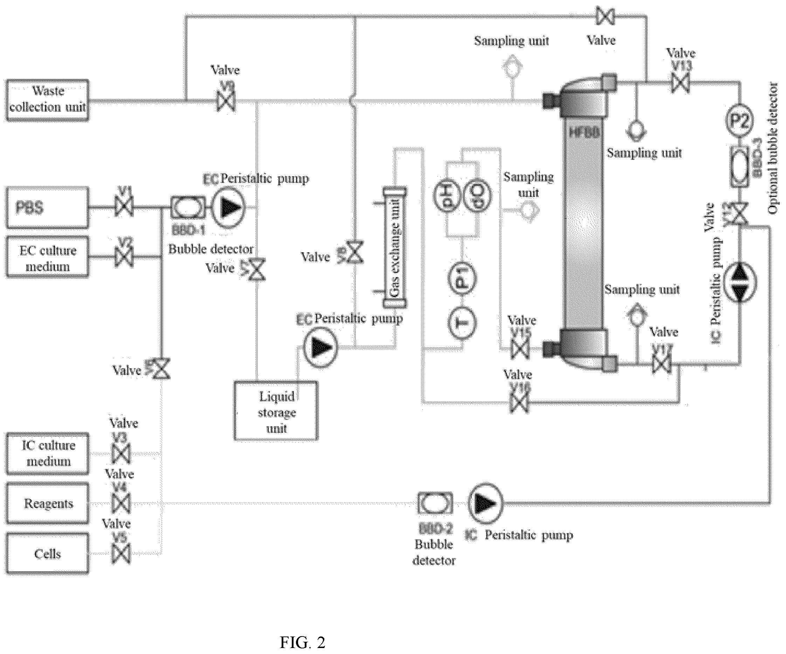

FIG. 2 shows a schematic diagram of a system for producing a target substance according to an embodiment. Referring to FIG. 2, the culture device in the system of the present disclosure is a hollow fiber tube reactor (HFBB). The first culturing solution supply device includes a culture medium supply unit (i.e., EC culture medium) and a pretreatment solution supply unit (i.e., PBS) as well as a liquid storage unit. The second culturing solution supply device includes a culture medium supply unit (i.e., IC culture medium) and a pretreatment solution supply unit (i.e., Reagents). The PBS supply unit and the EC culture medium supply unit are in communication with the hollow fiber tube reactor through a pipeline. The IC culture medium supply unit and the reagent supply unit are in communication with the hollow fiber tube reactor through another pipeline. The culture substance supply unit supplies the culture substance to the growth zone of the hollow fiber tube reactor. A plurality of valves (V1, V2, V5, V15, V16), a bubble detector, a plurality of peristaltic pumps, a temperature sensor, a pressure sensor, a pH sensor, a dissolved oxygen sensor and a plurality of sampling units are disposed between the first culturing solution supply device and the hollow fiber tube reactor. A plurality of valves (V3, V4, V5, V12, V13, V17), a plurality of bubble detectors, a plurality of peristaltic pumps, a pressure sensor and a plurality of sampling units are disposed between the second culturing solution supply device and the hollow fiber tube reactor. The system further includes a waste liquid collection unit. A plurality of valves (V9) and a sampling unit are disposed between the waste liquid collection unit and the hollow fiber tube reactor.

In another aspect, the embodiments of the present disclosure provide a method for producing a target substance. The method can be performed using the system described in any of the embodiments described above.

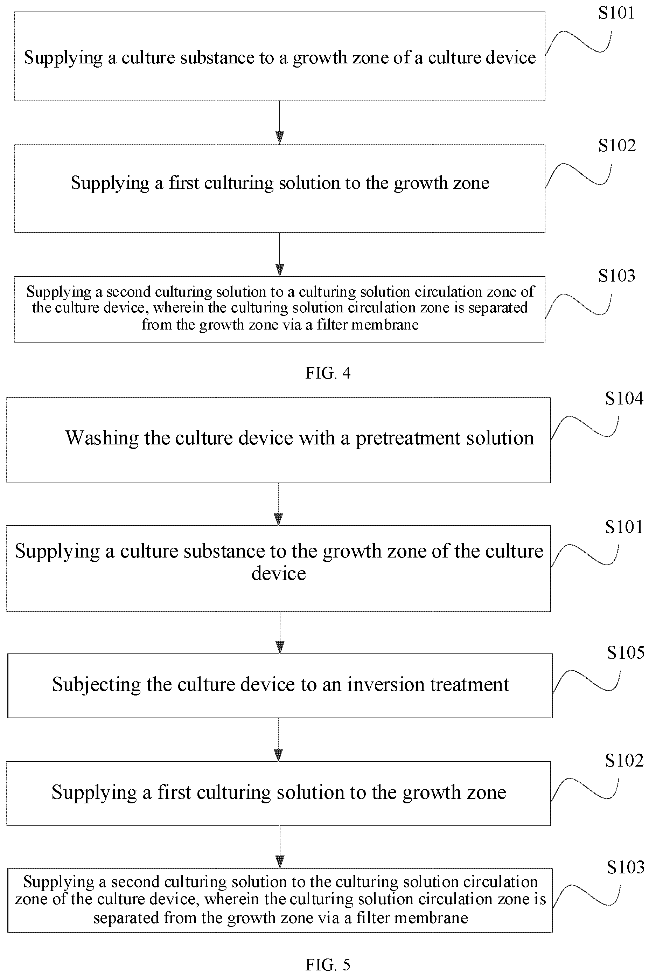

FIG. 4 shows a flow chart of a method for producing a target substance according to an embodiment of the present disclosure. Referring to FIG. 4, the method includes S101 to S103:
S101: supplying a culture substance to the growth zone of the culture device;
S102: supplying a first culturing solution to the growth zone; and
S103: supplying a second culturing solution to the culturing solution circulation zone of the culture device, wherein the culturing solution circulation zone is separated from the growth zone via a filter membrane.

In an embodiment of the present disclosure, the method includes S104: washing the culture device with a pretreatment solution before step a).

In a specific embodiment, the growth zone and the culturing solution circulation zone are washed multiple times with a pretreatment solution to fully remove impurities. The pretreatment solution can be a buffer solution, such as sterile water, normal saline, phosphate buffer saline (PBS), 4-hydroxyethylpiperazineethanesulfonic acid (HEPES) buffer, tris(hydroxymethylaminomethane) (Tris) buffer, etc.

In a specific embodiment, the pretreatment solution can also be a coating solution. In a specific embodiment of the present disclosure, the coating solution is an aqueous solution or a salt buffer solution containing one or more of fibronectin, vitronectin, osteopontin, collagen, gelatin, laminin or polylysine. Specifically, the coating solution is a mixture of 5 mL of deionized water containing 1 mg to 10 mg of fibronectin and 45 mL of phosphate buffer saline. For example, the coating solution is a mixture of 5 mL of deionized water containing 5 mg of fibronectin and 45 mL of phosphate buffer saline.

In a specific embodiment, the growth zone is pre-coated before inoculating the culture substance, such as cells. More specifically, the membrane surface of the filter membrane corresponding to the growth zone is pre-coated with the coating solution to enhance the adhesion ability of cells.

In an embodiment of the present disclosure, the pre-coating treatment includes:
washing the growth zone and the culturing solution circulation zone with a buffer solution,
removing the buffer solution in the growth zone and optionally in the culturing solution circulation zone, and
injecting a prepared coating solution into the growth zone and maintaining for 6 to 24 hours.

In a specific embodiment, the prepared coating solution is injected into the growth zone and maintained for 6 to 24 hours, for example, 6 hours, 7 hours, 8 hours, 9 hours, 10 hours, 11 hours, 12 hours, 13 hours, 14 hours, 15 hours, 16 hours, 17 hours, 18 hours, 19 hours, 20 hours, 21 hours, 22 hours, 23 hours or 24 hours to allow the fibronectin to be fully attached to the hollow fiber membrane.

Using the coating solution, the culture substance such as cells can be more evenly adhered to the membrane surface of the growth zone, thereby significantly reducing cell aggregation and enabling cells to grow efficiently.

In an embodiment of the present disclosure, the method further includes: S105: subjecting the culture device to an inversion treatment to achieve sufficient adhesion of the culture substance, such as cells, after step a) and before step b).

The inversion treatment includes inverting the culture device 6 to 10 times at a frequency of 20 to 40 minutes per time. For example, the culture device is inverted once every 30 minutes for 8 times in total, so that adhesion of the culture substance, such as cells, is lasted for 4.5 hours.

The specific inversion treatment can be beneficial to the adhesion of the culture substance, such as cells, thereby significantly reducing aggregation and death of the cells, and advantageously promoting the growth of cells at a higher growth rate and the efficient production of cells and target metabolites.

In an embodiment of the present disclosure, the first culturing solution is supplied to the growth zone at a flow rate of about 0.05 to 0.30 mL/min, such as 0.05 to 0.25 mL/min, such as 0.05 to 0.20 mL/min, such as 0.05 to 0.15 mL /min, such as 0.05 to 0.10 mL/min. In one embodiment, the first culturing solution is supplied to the growth zone at a flow rate of about 0.08 to 0.12 mL/min. In another embodiment, the first culturing solution is supplied to the growth zone at a flow rate of 0.05 mL/min, 0.06 mL/min, 0.07 mL /min, 0.08 mL /min, 0.09 mL/min, 0.10 mL/min, 0.11 mL/min, or 0.12 mL/min.

In an embodiment of the present disclosure, the second culturing solution is supplied to the culturing solution circulation zone at a flow rate of about 50 to 500 mL/min, such as 50 to 150 mL/min, such as 50 to 120 mL/min, such as 50 to 100 mL/min, such as 150 to 500 mL/min, such as 150 to 450 mL/min, such as 150 to 400 mL/min, such as 150 to 350 mL/min, such as 150 to 300 mL/min, such as 150 to 250 mL/min, such as 150 to 200 mL/min. In one embodiment, the second culturing solution is supplied to the culturing solution circulation zone at a flow rate of about 100 mL/min to 300 mL/min. In another embodiment, the second culturing solution is supplied to the culturing solution circulation zone at a flow rate of 100 mL/min, 110 mL/min, 120 mL / min, 150 mL/min, 180 mL/min, 200 mL/min, 250 mL/min, or 300 mL/min.

The first culturing solution is supplied to the growth zone at a relatively low flow rate, and the second culturing solution is supplied to the culturing solution circulation zone at a relatively high flow rate, thereby, based on the flow rate difference, supplying the nutrients and oxygen required by the culture substance to the growth zone through the membrane, and taking the metabolic waste away as the culturing solution in the culturing solution circulation zone rapidly flows, while ensuring that the culture substance grows stably with sufficient nutrients.

In an embodiment of the present disclosure, during the culture period, the concentration of the target substance in the growth zone, along with the contents of lactic acid and glucose in the growth zone and the culturing solution circulation zone, are measured at a frequency of 1 to 4 days/time, for example, 1 to 3 days /time, for example, 1 to 2 days /time, for example, 1 day/time. During the culture period, the pH, dissolved oxygen and intracavitary pressure in the growth zone and the culturing solution circulation zone are monitored online in real time. When the lactic acid level reaches 4 mM, more frequent measurement is performed, and the culturing solution supply scheme is adjusted in real time according to the measurement results. Thus, the culture substance is guaranteed to grow under optimal culture conditions.

In the embodiment of the present disclosure, the target substance, such as target cells and/ or their target metabolites, are collected by the sampling unit via the first inlet and/or the first outlet.

In an embodiment, the target substance can be the culture substance itself or a metabolite produced by the culture substance. In a specific embodiment, the culture substance includes but is not limited to mesenchymal stem cells. The target substance can be extracellular vesicles. In a specific embodiment, the extracellular vesicles are exosomes.

In a specific embodiment, the first culture medium is the most suitable culture medium for culturing the culture substance. In a specific embodiment, the culture substance is mesenchymal stem cells. Therefore, the first culture medium is MSC culture medium.

In the embodiments of the present disclosure, the culture substance includes but is not limited to bacteria, adherent cells such as mesenchymal stem cells, fibroblasts, endothelial cells, etc., or suspended cells such as immune cells, hematopoietic stem cells, neural stem cells, etc. The culture substance can also be a microcarrier loaded with cells or encapsulated with cells.

In the embodiment of the present disclosure, the second culture medium provides the culture substance in the growth zone with nutrients and oxygen required for growth. The second culture medium and the first culture medium can be the same or different. The composition of the second culture medium can be adjusted as needed.

In a specific embodiment, the second culture medium can include, but is not limited to, DMEM culture medium, MSC culture medium, cell growth promoting factors, fetal bovine serum, sugars, amino acids, vitamins, inorganic ions and trace elements, etc. Thus, sufficient nutrients can be provided for the culture substance in the growth zone.

In an embodiment of the present disclosure, the filter membrane can be a microfiltration membrane, an ultrafiltration membrane or a nanofiltration membrane. In a specific embodiment, the filter membrane is a hollow fiber tube.

In a specific embodiment, the growth zone is the outer space of the hollow fiber tube, and the culturing solution circulation zone is the inner space of the hollow fiber tube.

In a specific embodiment, the growth zone is the inner space of the hollow fiber tube, and the culturing solution circulation zone is the outer space of the hollow fiber tube.

According to an embodiment of the present disclosure, the hollow fiber tube has a pore size of not more than about 500 nm. In a specific embodiment, the hollow fiber tube has a pore size of not more than about 40 nm. In a specific embodiment, the hollow fiber tube has a pore size of not more than about 80 nm. In a specific embodiment, the hollow fiber tube has a pore size of not more than about 100 nm. In a specific embodiment, the hollow fiber tube has a pore size of not more than about 120 nm. In other embodiments, the pore size of the hollow fiber tube can vary according to the size of the target substance desired to be produced.

In a specific embodiment, in case where the target substance is exosomes, the pore size of the filter membrane of the culture device is set to be less than about 40 nm. The mesenchymal stem cells are inoculated at a cell density of about 1 ×10⁶ cells/ ml to about 1 ×10⁸ cells/ ml. The first culture medium is supplied to the growth zone at a flow rate of about 0.01 mL/min, and the second culture medium is supplied to the culturing solution circulation zone at a flow rate of about 100 mL/min to about 300 mL/min. The culture period is several minutes, several hours, several days, or even several weeks, such as 30 minutes, 1 hour, 5 hours, 10 hours, 15 hours, 24 hours, 48 hours, 3 to 13 days, or even longer. The first culture medium and the second culture medium can be MSC culture medium. Thus, high-quality exosomes can be obtained with high yield.

The system and method of the present disclosure can be used to culture any substance suitable for culturing in the system, not limited to the mesenchymal stem cells shown below as examples, which includes human mesenchymal stem cells, animal mesenchymal stem cells, etc.

### Examples

### Example 1. Determination for Mesenchymal Stem Cells

In this example, mesenchymal stem cells were cultured by using the method as follows.

### 1.1 Preparation of culture medium and cell culture consumables for cell culturing

Pretreatment culturing solution: PBS containing 1% penicillin-streptomycin, DMEM culture medium containing 1% penicillin-streptomycin, and 4L MSC culture medium (i.e., MSC culture medium supplemented with 10% EV-depleted MSC culture medium and 1% penicillin-streptomycin).

Cell culture medium: The MSC cell culture medium was thawed in a 4°C refrigerator, centrifuged with an ultracentrifuge. The supernatant of the MSC cell culture medium was collected. The supernatant was filtered through a 0.22 µm filter and placed in a 4°C refrigerator for later use.

The cell culture consumables and pipes were sterilized in an autoclave. The sterilized cell culture consumables were placed in an oven to dry out the moisture. At the same time, several 2 mL ultracentrifuge tubes were sterilized in the autoclave for 15 minutes and placed in an oven to dry for later use.
1.2 Pre-coating treatment of hollow fiber tube reactor: A buffer supply unit into which phosphate buffer saline (PBS) was injected was used to supply phosphate buffer saline (PBS) into the space inside the membrane (i.e., culturing solution circulation zone) of the hollow fiber tube reactor. The phosphate buffer saline was then supplied to the space outside the membrane (i.e., growth zone) of the hollow fiber tube reactor. Afterward, the liquid in the growth zone was drained as much as possible. A coating solution (a solution of 5 mL deionized water containing 5 mg fibronectin and 45 mL of PBS) was prepared using a 60 mL luer-lock syringe and loaded through the inlet corresponding to the growth zone. The coating scheme was carried out, and the membrane of the growth zone was allowed to be coated with fibronectin overnight, and then the pre-coated hollow fiber tube reactor was placed in a 4°C refrigerator for storage.
1.3 System assembly: The sterilized consumables, the coated hollow fiber tube reactor, the culturing solution supply devices, the peristaltic pumps, the coating box FX10, the medium bag, the sensor group and the pipelines were assembled. PBS buffer, followed by DMEM culture medium, and finally MSC culture medium were loaded into the culturing solution circulation zone and growth zone of the hollow fiber tube reactor, thereby achieving complete culture medium washing of the hollow fiber tube reactor and the pipeline. Afterwards, the culture medium in the storage bottle was discarded and replaced with fresh MSC culture medium.
1.4 Seeding of MSCs to be cultured: The pre-proliferated MSCs (approximately 1×10⁶ cells to approximately 1×10⁸ cells) were digested in a flask, resuspended in approximately 40 to 50 mL of MSC culture medium (EV-depleted), and then transferred to a 60 mL Luer lock syringe.

The prepared mesenchymal stem cells to be cultured were inoculated in the growth zone of the hollow fiber bioreactor. Specifically, the hollow fiber tube reactor was placed horizontally. The pipes at the inlet and outlet of the growth zone were clamped with hemostats, while the valves V17 and V13 at both ends of the culturing solution circulation zone were closed and the pipes at the inlet and outlet of the culturing solution circulation zone were clamped, so that the culturing solution circulation between the growth zone and the culturing solution circulation zone was stopped. The prepared cell suspension was injected into the growth zone via a syringe through the inlet of the growth zone. At the same time, the culturing solution replaced by the cell suspension was extracted at the outlet of the growth zone using another syringe. Subsequently, the culturing solution extracted from the outlet was injected into the inlet, and the replaced cell-containing mixed solution was extracted from the outlet with a syringe. Thus, the extraction through the syringe at the inlet and outlet was repeated 5 to 6 times until the turbidity of the cell mixture inside the two syringes was approximately the same. The hemostat at the inlet or outlet of the culturing solution circulation zone was released. The cell mixture in the syringe was injected into the growth zone.
1.5 The hollow fiber tube reactor was inverted regularly to allow the cells to adhere better and more evenly. Specifically, maintaining the pipes at the inlet and outlet of the growth zone closed, the hollow fiber tube reactor was inverted every 30 minutes at an ambient temperature of 37°C for 8 times, allowing the cells to adhere to the hollow fiber membrane for 4.5 hours. During this period, the culturing solution was circulated in the culturing solution circulation zone at a flow rate of 100 mL/min.
1.6 Cells were cultured in an automated mode, with the culturing solution and oxygen circulated through the pipes and supplied to the cells in the growth zone, and the waste products removed with the circulation. The whole system automatically replenished the fluid. Specifically, the hemostats at the inlet and outlet of the growth zone were released, and the culturing solution was supplied to the growth zone at a flow rate of 0.1 mL/min to achieve the automatic circulation mode.
1.7 Regular monitoring of cell metabolism indicators. Specifically, the glucose and lactic acid contents and cell level of the circulating culturing solution were measured at regular intervals. At beginning of the culturing, the measurement was performed once every 2 days. When the lactic acid level reaches 4 mM, more frequent measurement is performed, and the culturing solution supply scheme is adjusted in real time according to the measurement results. Sampling and testing are specifically as follows. The hollow fiber tube reactor was upright placed, with the sampling port corresponding to the inlet of the growth zone facing upward. Using syringes, 2 mL of crude culturing solution was drawn at the sampling port of the growth zone and the sampling port of the culturing solution circulation zone, respectively. 0.5 mL of crude culture solution was used to measure lactic acid and glucose, and 1.5 mL of crude culturing solution was used to measure the particle number of cells and protein concentration.

After the culturing solution circulation zone was sampled, the IC peristaltic pump corresponding to the culturing solution circulation zone was turned off, valve V15 was closed, the pipe corresponding to the outlet of the culturing solution circulation zone was clamped with hemostat, and the EC peristaltic pump was turned off.
1.8 The generated mesenchymal stem cells (MSCs) was collected and the cells and culturing solution were sampled for testing. Specifically, the sampling port at the inlet of the growth zone (on the fluid replenishment side) was connected to a 0.22 µm filter, and then connected to a 60 mL syringe. The syringe was used to inject filtered air into the growth zone. At the same time, a syringe at the sampling port at the outlet of the growth zone (away from the fluid replenishment side) was used to extract nearly 40 mL of the replaced MSC-containing culturing solution. The obtained MSC-containing culture solution was collected in a storage bottle and mixed. 2 mL of the mixed solution was retained for the measurement of lactic acid, glucose, and the particle number of cells and protein concentration.
1.9 The flow rate of the peristaltic pump used to feed liquid to the growth zone was reset. The fresh culturing solution was injected from the inlet into the growth zone, and a syringe was used to extract air from the sampling port at the outlet of the growth zone. When the growth zone was filled with the culturing solution again, the flow rate of the peristaltic pump for feeding liquid into the growth zone was reset to 0.2 mL/min.
1.10 The cell culture mode was restored after the cultured mesenchymal stem cells (MSCs) were collected. Specifically, the hemostat at the outlet of the culturing solution circulation zone was released, valve V15 was opened, and the IC culturing solution circulation was reset.

At the same time, cells with the same cell inoculating density were cultured in a T175 flask as a cell reactor by using the same cell growth culturing solution as a comparative example. The specific experimental results are shown below.

### 1.11 Determination of total viable cells

During the entire culture period, a certain amount of cell culture substance was collected from the growth zone of the culture device of the present system and from the T175 flask respectively every day, from the start of culturing (day 0) to day 8 to determine the total number of living cells by using a microscope.

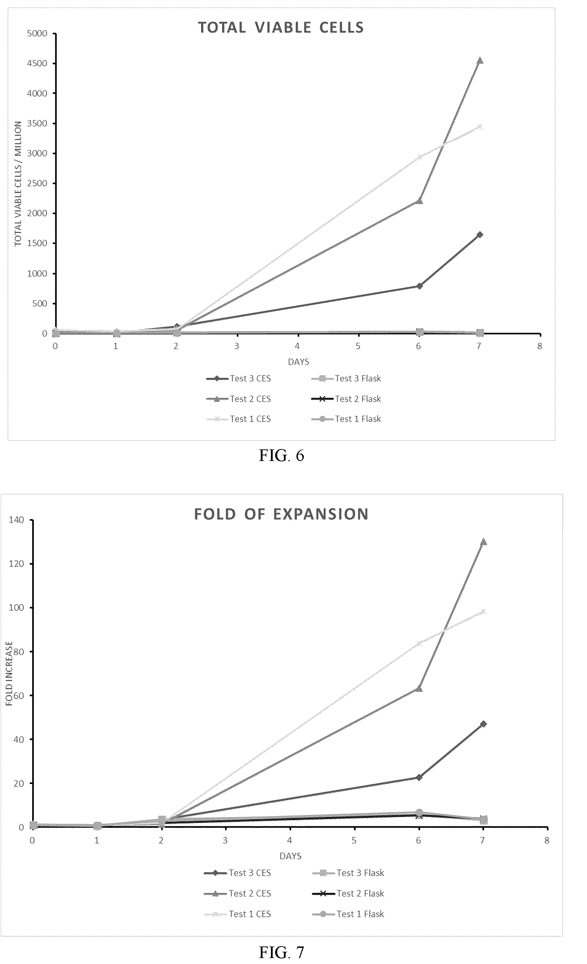

The results of cell counting are shown in FIG. 6. The results show that the cells in the growth zone of the system of the present disclosure grew exponentially, and the number of cells can reach more than 400 million cells. Compared with those of the T175 flask as the cell reactor, the number of cells produced by the present system was 10 more folds higher than the number of cells produced by the T175 flask, indicating that the system of the present disclosure has a significantly higher cell production efficiency.

### 1.12 Determination of the expansion fold of viable cells

The cell expansion fold was obtained by dividing the total number of viable cells obtained each day from day 0 to day 8 by the starting cell number.

The results of cell expansion fold are shown in FIG. 7. The results show that, compared with the T175 flask as the cell reactor, the expansion fold of cells produced by the system of the present disclosure was 10 to 30 folds higher than the expansion fold of cells produced by the T175 flask, indicating that the system of the present disclosure has a significantly higher cell production efficiency.

### 1.13 Determination of cell viability

The cell viability was calculated by dividing the total number of viable cells obtained each day from day 0 to day 8 by the total number of cells obtained (including viable cells and dead cells).

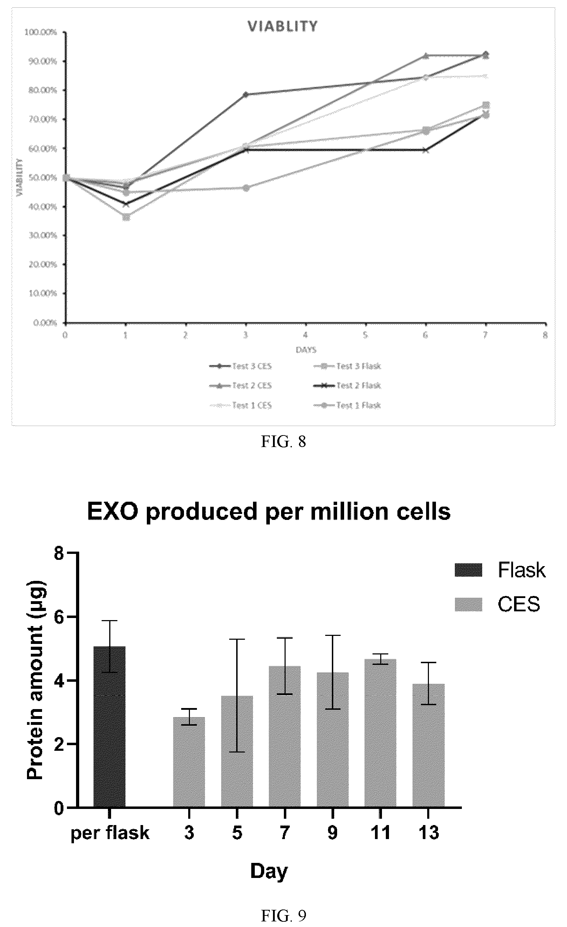

The results of cell viability are shown in FIG. 8. The results show that the cell viability obtained by the system of the present disclosure is comparable to that obtained by using the T175 flask as the cell reactor. It can be seen that using the system of the present disclosure as a cell reactor does not affect the viability of the cells.

### Example 2. Production of mesenchymal stem cells (MSCs) and extracellular vesicles (EVs)

In this example, human mesenchymal stem cells and target metabolite extracellular vesicles produced by the cells were further produced according to the method of Example 1, except that the steps 1.8 to 1.10 of Example 1 were replaced by the following design: 1.8-1 The generated mesenchymal stem cells (MSCs) and extracellular vesicles (EVs) were collected and the cells and culturing solution were sampled for detection. Specifically, the sampling port at the inlet of the growth zone (i.e., at the fluid replenishment side) was connected to a 0.22 µm filter, and then connected to a 60 mL syringe. The syringe was used to inject filtered air into the growth zone. At the same time, a syringe at the sampling port at the outlet of the growth zone (i.e., at a side away from the fluid replenishment side) was used to extract nearly 40 mL of the replaced MSC-containing culturing solution. The obtained MSC-containing culturing solution was collected in a storage bottle and mixed. 2 mL of the mixed solution was retained for the measurement of lactic acid, glucose, and the particle number of cells and protein concentration.
1.9-1 The flow rate of the peristaltic pump used to feed liquid to the growth zone was reset. The culturing solution was injected into the inlet of the growth zone, and a syringe was used to extract air from the sampling port at the outlet of the growth zone. When the growth zone was filled with culturing solution again, the flow rate of the peristaltic pump used to feed liquid to the growth zone was reset to 0.2 mL/min.
1.10-1 The cell culture mode was restored after the cultured mesenchymal stem cells (MSCs) and extracellular vesicles (EVs) were collected. Specifically, the hemostat at the outlet of the culturing solution circulation zone was released, valve V15 was opened, and the IC culturing solution circulation was reset.

The experimental test process for verification of the design of this example was entrusted to the Oxford University Laboratory. The test results of this example are as follows:

### 2.1 Determination of exosome production per million cells

In this example, the content of exosomes produced by mesenchymal stem cells cultured in the system of the present disclosure and in the T175 flask according to the method as described in Example 2 was measured respectively.

When the exosome yield per million cells was expressed as protein amount, the results are shown in FIG. 9. The results show that the exosomes per million mesenchymal stem cells harvested in the system of the present disclosure on day 3 was slightly lower than the yield of the exosomes produced in the T175 flask. From day 5 onwards, there was no significant difference between the yield of the exosomes per million mesenchymal stem cells harvested in the system of the present disclosure and the yield of the exosomes produced in the T175 flask. The results show that the system of the present disclosure can be used to produce exosomes.

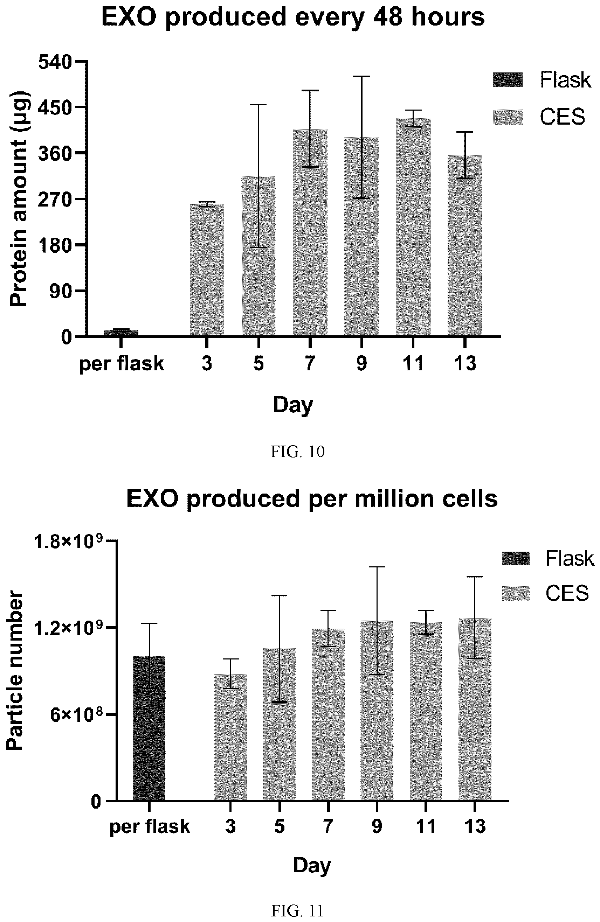

When the exosome yield per million cells was expressed as particle numbers, the results are shown in FIG. 11. The results show that the exosome yield per million mesenchymal stem cells harvested in the system of the present disclosure is even higher than the yield of the exosomes produced in the T175 flask. The results show that the system of the present disclosure can be used to produce exosomes.

### 2.2 Determination of the total amount of exosomes produced every 48 hours

In this example, the total amount of exosomes produced every 48 hours was measured. When the total amount of exosomes produced every 48 hours was expressed as protein amount, the results are shown in FIG. 10. The results show that the total amount of exosomes harvested every 48 hours in the system of the present disclosure was significantly higher than the total amount of exosomes obtained every 48 hours in the single T175 flask with the same cell culture volume during the entire growth period of 13 days. The system of the present disclosure provides cells with a larger growth zone and can accommodate more cells. During the entire growth period of 13 days, the yield of exosomes harvested in the system of the present disclosure was 18 more folds higher than the yield of exosomes harvested from the T175 flask.

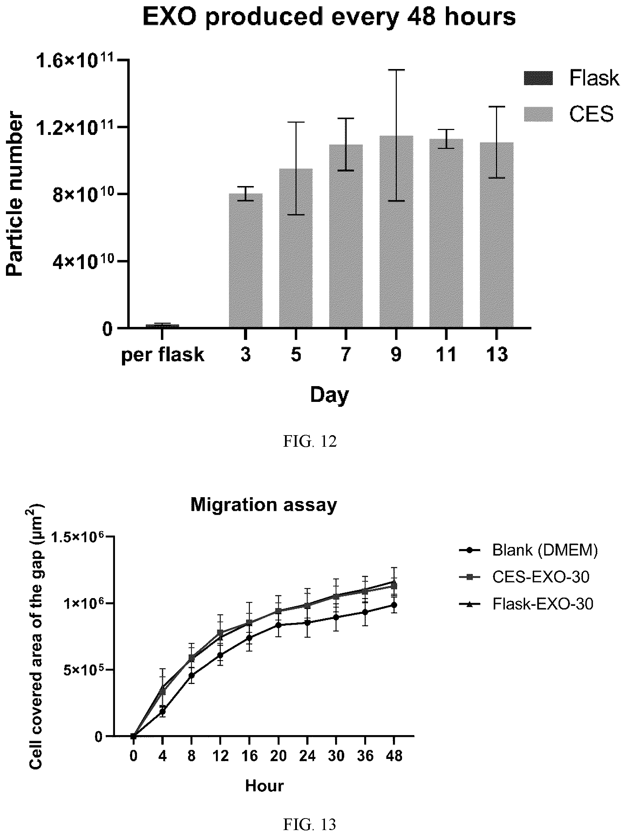

When the total amount of exosomes produced every 48 hours was expressed as particle numbers, the results are shown in FIG. 12. The results show that the total amount of exosomes harvested every 48 hours in the system of the present disclosure was significantly higher than the total amount of exosomes obtained every 48 hours in the single T175 flask with the same cell culture volume during the entire growth period of 13 days. During the entire growth period of 13 days, the yield of exosomes harvested in the system of the present disclosure was 250 more folds higher than the yield of exosomes harvested from the T175 flask.

### Example 3. Determination of the biological activity of produced exosomes

The production of exosomes from human mesenchymal stem cells according to the method of Example 2 and measurement of their biological activity were entrusted to Oxford University.

### 3.1 Cell migration assay

The exosomes produced in the system of the present disclosure and the exosomes produced in T175 flask were each co-cultured with normal human fibroblasts.

The experimental results of fibroblast migration are shown in FIG. 13. The experimental results show that both the exosomes produced in the system of the present disclosure and the exosomes produced in T175 flask promote the migration of fibroblasts, with comparable promotion effect, indicating that the exosomes produced in the system of the present disclosure and the exosomes produced in T175 flask have comparable biological activities.

### 3.2 Cell proliferation assay

In this example, the exosomes produced in the system of the present disclosure and the exosomes produced in T175 flask were each co-cultured with normal human fibroblasts. By using T175 flask, the obtained exosomes can have a maximum concentration of only 30 µg /mL, while the concentration of exosomes obtained by the present system can be up to 70 µg /mL. The proliferation effect of fibroblasts under the action of exosomes was determined using a CCK-8 cell viability assay.

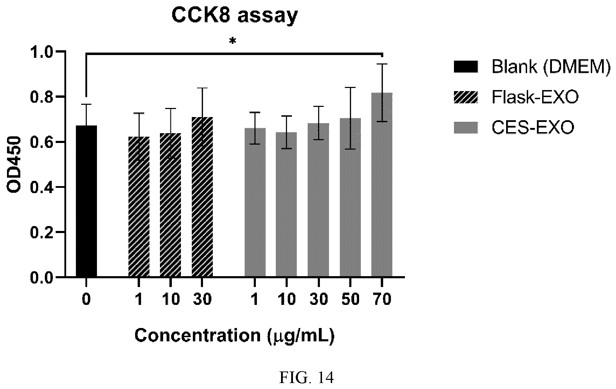

The results of cell proliferation are shown in FIG. 14. The experimental results show that the exosomes produced in the system of the present disclosure and the exosomes produced in T175 flask can both effectively promote the proliferation of fibroblasts.

In the specification of the present disclosure, it is to be understood that terms such as "central", "longitudinal", "lateral", "length", "width", "thickness", "upper", "lower", "front", "rear", "left", "right", "vertical", "horizontal", "top", "bottom", "inner", "outer", "clockwise", "counterclockwise", "axial", "radial", and "circumference" refer to the orientations and location relations which are the orientations and location relations illustrated in the drawings, and for describing the present disclosure and for describing in simple, and which are not intended to indicate or imply that the device or the elements are disposed to locate at the specific directions or are structured and performed in the specific directions, which could not to be understood to the limitation of the present disclosure.

The terms such as "first" and "second" are used herein for purposes of description and are not intended to indicate or imply relative importance or significance or to imply the number of indicated technical features. Thus, the feature defined with "first" and "second" can explicitly or implicitly include at least one of the features. In the description of the present disclosure, "a plurality of" means two or more than two, such as two, three, etc., unless specified otherwise.

In the present disclosure, unless specified or limited otherwise, the terms "mounted", "connected", "coupled", "fixed" and the like are used broadly, and can be described for, for example, fixed connections, detachable connections, or integral connections; can also be described for mechanical or electrical connections or communications with each other; can also be described for direct connections or indirect connections via intervening structures; can also be described for inner communications or interactive relationship of two elements, unless otherwise explicitly limited. For those of ordinary skill in the art, the specific meanings of the above terms in the present disclosure can be understood according to specific circumstances.

In the present disclosure, unless specified or limited otherwise, a structure in which a first feature is "on" or "below" a second feature can include an embodiment in which the first feature is in direct contact with the second feature, and can also include an embodiment in which the first feature and the second feature are contacted via an intermediate medium. Furthermore, a first feature "on", "above", or "on top of" a second feature can include an embodiment in which the first feature is right or obliquely "on", "above", or "on top of" the second feature, or just means that the first feature is at a height higher than that of the second feature; while a first feature "below", "under", or "on bottom of" a second feature can include an embodiment in which the first feature is right or obliquely "below", "under", or "on bottom of" the second feature, or just means that the first feature is at a height lower than that of the second feature.

Reference throughout this specification to "an embodiment", "some embodiments", "one embodiment", "another example", "an example", "a specific example", or "some examples", means that a particular feature, structure, material, or characteristic described in connection with the embodiment or example is included in at least one embodiment or example of the present disclosure. Thus, the appearances of these phrases throughout this specification are not necessarily referring to the same embodiment or example of the present disclosure. Furthermore, the particular features, structures, materials, or characteristics can be combined in any suitable manner in one or more embodiments or examples.

The technical features of the above-described embodiments can be arbitrarily combined. To make the specification concise, not all possible combinations of the technical features in the above-described embodiments are described. However, they should be considered to be within the scope of this specification as long as there is no contradiction in the combination of these technical features.

The foregoing embodiments only show several implementations of the present disclosure and are described in detail, but they are not to be construed as a limit to the patent scope of the present disclosure. It should be noted that a person of ordinary skill in the art can further make variations and improvements without departing from the ideas of the present disclosure, and the variations and improvements shall fall within the protection scope of the present disclosure. Therefore, the patent protection scope of the present disclosure is subject to the protection scope of the appended claims.

## Claims

1. A system for producing a target substance, comprising:
a. a culture device configured to be divided into a growth zone and a culturing solution circulation zone via a filter membrane, and comprising a first inlet, a first outlet, a second inlet and a second outlet,
b. a first culturing solution supply device configured to be in fluid communication with the growth zone via the first inlet and the first outlet,
c. a second culturing solution supply device configured to be in fluid communication with the culturing solution circulation zone via the second inlet and the second outlet, and
d. a culture substance supply unit configured to be in fluid communication with the growth zone via the first inlet.

2. The system according to claim 1, further comprising:
e. a plurality of perfusion pumps disposed between the culture device and the first culturing solution supply device and/or disposed between the culture device and the second culturing solution supply device.

3. The system according to claim 1 or 2, wherein a first valve is disposed between the first culturing solution supply device and the first inlet, and a second valve is disposed between the first culturing solution supply device and the first outlet.

4. The system according to claim 1 or 2, wherein a third valve is disposed between the second culturing solution supply device and the second inlet, and a fourth valve is disposed between the second culturing solution supply device and the second outlet.

5. The system according to claim 1 or 2, wherein the culture substance supply unit is in fluid communication with the first culturing solution supply device via a fifth valve, and
the first culturing solution supply device is in fluid communication with the second culturing solution supply device via a sixth valve.

6. The system according to claim 1 or 2, further comprising a plurality of sensors, wherein the sensors are configured to perform sensing in a flow path between the culture device and the first culturing solution supply device, and/or
the sensors are configured to perform sensing in a flow path between the culture device and the second culturing solution supply device.

7. The system according to claim 1 or 2, further comprising an oxygenator, wherein the oxygenator is configured to supply oxygen to a channel between the second culturing solution supply device and the culture device.

8. The system according to claim 1 or 2, further comprising a plurality of sampling units, wherein the plurality of sampling units are in fluid communication with one or more of the first inlet, the first outlet, the second inlet or the second outlet.

9. The system according to claim 1 or 2, further comprising a waste collection unit, wherein the waste collection unit is configured to be in fluid communication with the culture device via the first outlet or the second outlet.

10. The system according to claim 1 or 2, wherein the first culturing solution supply device comprises a first culture medium supply unit and a first pretreatment solution supply unit, and/or
the second culturing solution supply device comprises a second culture medium supply unit and a second pretreatment solution supply unit.

11. The system according to claim 1 or 2, wherein the filter membrane is a hollow fiber tube, the growth zone is an outer space of the hollow fiber tube, and the culturing solution circulation zone is an inner space of the hollow fiber tube; or
the growth zone is an inner space of the hollow fiber tube, and the culturing solution circulation zone is an outer space of the hollow fiber tube.

12. The system according to claim 1 or 2, wherein a surface of the filter membrane corresponding to the growth zone is treated with a coating solution.

13. The system according to claim 12, wherein the coating solution is an aqueous solution or a salt buffer solution containing one or more of fibronectin, vitronectin, osteopontin, collagen, gelatin, laminin, or polylysine.

14. A method for producing a target substance, comprising:
a) supplying a culture substance to a growth zone of a culture device;
b) supplying a first culturing solution to the growth zone; and
c) supplying a second culturing solution to a culturing solution circulation zone of the culture device, wherein the culturing solution circulation zone is separated from the growth zone via a filter membrane.

15. The method according to claim 14, wherein the first culturing solution is supplied to the growth zone at a flow rate of 0.05 to 0.30 mL/min, and/or
the second culturing solution is supplied to the culturing solution circulation zone at a flow rate of 50 to 500 mL/min.

16. The method according to claim 14 or 15, further comprising washing the culture device with a pretreatment solution before step a).

17. The method according to claim 14 or 15, further comprising subjecting the culture device to an inversion treatment after step a) and before step b),
wherein the inversion treatment comprises inverting the culture device 6 to 10 times at a frequency of 20 to 40 minutes per time.

18. The method according to claim 14 or 15, wherein during the culture period, a concentration of the target substance in the growth zone and contents of lactic acid and glucose in the growth zone and the culturing solution circulation zone are detected at a frequency of 1 to 4 days/time, and/or
a pH value, dissolved oxygen, and intracavitary pressure in the growth zone and the culturing solution circulation zone are monitored online in real time.

19. The method according to claim 14 or 15, wherein the culture substance is mesenchymal stem cells, and the target substance is extracellular vesicles.

20. The system or method according to any one of claims 1 to 19, wherein the culture substance and the target substance comprise human cells, animal cells, human extracellular vesicles and/or animal extracellular vesicles.
